# EUROPEAN PATENT APPLICATION

(11) **EP 2 572 637 A2**
(43) Date of publication of application: **27.03.2013**
(21) Application number: 11783689.0
(22) Date of filing: 15.04.2011
(51) Int. Cl.: A61B 5/0482, A61B 5/0484, A61B 5/11

(54) **DEVICE FOR EXAMINING NERVE FUNCTION**

(30) Priority: 17.05.2010 KR 20100046093
(71) Applicant: Park, Jong-Eun, Seoul 135-500 (KR)
(72) Inventor: Park, Jong-Eun, Seoul 135-500 (KR)
(74) Representative: Grunert, Marcus
(86) International application number: PCT/KR2011/002718
(87) International publication number: WO 2011/145807

(57) **Abstract**

The present invention relates to a device for examining nerve function. In order to examine the function of a nerve of the human body, the device for examining nerve function of the present invention includes: at least one stimulator for stimulating the human body; and a stimulator-fixing member having a form corresponding to the hands or feet of the human body, wherein the stimulator is located at one side of the simulator-fixing member so as to fix the stimulator to the hands or feet of the human body. The device for examining nerve function of the present invention further includes at least one blood-circulation detection sensor fixed at the stimulator-fixing member. According to the present invention, since the degree of hypoesthesia is examined through the stimulation of the stimulator, test accuracy and reliability may be greatly improved and it is possible to systematically and actively determine a diagnosis and course of treatment for a patient having diabetes or spinal nerve damage, e.g. hypoesthesia.

## Description

### Technical Field

The present invention relates to a device for examining nerve function, and more specifically, to a device for examining nerve function which examines a sensation in the hands or the feet of a subject such as a patient having diabetes or spinal nerve damage, so as to check a disease condition, to prevent an accident, and to treat the disease.

### Background Art

In general, diabetes is a disease in which cells are unable to absorb sugars entered into the body and the blood sugar level increases, either because an enough amount of insulin that the body requires is not produced, or because the produced insulin does not work properly in the cells.

The diabetes may lead to serious complications, for example complications in large blood vessels or small blood vessels developed from hardening of the arteries in blood vessels. This may cause renal failure and require long-term hemodialysis, or cause a coronary or cerebrovascular disorder or retinal damage, and even cause necrosis of the foot due to blood supply shortage.

Especially, when a neurological complication is developed and the peripheral nerve function declines in the nervous system which connects nerves from the head through the body to the hands and the feet, sensation becomes dulled. Even though stones or sand come into the shoes, it is hard to feel this. In such a case, there was a problem that the symptoms were more exacerbated by a secondary bacterial infection when the wound is caused on the foot during activity. The neurological complication, of course, may occur in the hand because the nervous system is connected to the hand. But, it is more common to occur in the foot rather than in the hand.

The hypoesthesia first occurs in the feet in which blood circulation is not performed smoothly. Unless properly treating or preventing this, small blood vessels in the toes would be blocked and as a result the situation that the toes get rotten may occur.

This finally may cause a serious situation that the above-knee amputation should be performed when a necrosis area starting from the toes gradually spreads out above the knees. In this case, a diabetic patient would have worse blood circulation and this will lead to not only a serious complication, but also even death.

Therefore, it is very important for diabetic patients to check the hypoesthesia symptom in advance for proper treatment and prevention of wounds. In the conventional arts, in order to examine the hypoesthesia symptom of the diabetic patients, doctors just pricked the patient's feet with a needle-shaped mono filament, or vibrated a "U"-shaped tuning fork and brought it into contact with the patient's toe or sole. However, these methods have the following problems: the examination result depending on an inspector is unreliable; since the patient's sensation condition is determined through empirical methods that are not standardized and not expressed in numbers, the examination is not relatively accurate; and it is unable to systematically check how much the patient's hypoesthesia has progressed, a specific area of the hypoesthesia, etc.

In the meanwhile, because paresthesia also happens to patients with spinal nerves damaged due to a spine disease or a spine accident and they show the same symptoms as discussed above, the similar examination is conducted for them like diabetic patients. Therefore, it is very important for those patients to exactly pinpoint the damaged area of nerves and the degree of the damage for selecting a proper treatment method such as emergency surgery.

### DISCLOSURE OF THE INVENTION

### PROBLEM TO BE SOLVED BY THE INVENTION

In order to solve the above-mentioned problems, an object of the present invention is to provide a device for examining nerve function capable of: greatly improving accuracy and reliability of the examination using a standardized stimulator and a standardized stimulator fixing member; expressing as a number the degree of hypoesthesia or the status of hypoesthesia through the examination for the patient's nerve function; exactly determining the specific position of the patients' hypoesthesia so as to systematically and actively determine a diagnosis and treatment course for the patients having hypoesthesia for example those suffering diabetes; and especially detecting the state of blood circulation to check the position of hypoesthesia.

Further, another object of the present invention is to provide a device for examining nerve function capable of: accurately adjusting the degree of stimulation by automatically moving a pressurizer having an actuator; and recording and checking the degree of hypoesthesia in real time and for a long period of time with a subject wearing an elastic clothing, stimulation gloves or stimulation boots in practical life.

In particular, another object of the present invention is to provide a device for examining nerve function capable of: checking if a subject can feel pain caused by applying pressure, and if a subject can distinguish positions among multiple pressure points (positions of pressure points), and if a subject can detect vibration, if a subject can detect change in temperature, and if a subject can distinguish difference in the applied pressure, through the pain caused by the applied pressure; and checking the state of blood circulation through oxygen saturation or temperature of the hypoesthesia area. In addition, the present invention aims to provide a device for examining nerve function capable of not only proceeding with the process of measuring the above-mentioned capabilities step by step but also reviewing the measured capabilities and the state of blood circulation in an audio or video message or in a printed material.

### MEANS FOR SOLVING PROBLEM

In order to achieve the above-described objects, the device for examining nerve function according to the present invention includes at least one stimulator for stimulating the human body to examine nerve function in the human body; and a stimulator fixing member formed in the shape corresponding to the hands or the feet of the human body, in which the stimulator is located at one side of the stimulator fixing member so as to fix the hands or the feet of the human body.

The stimulator may include at least one of a pressurizer for pressurizing the skin to provide stimulation and a vibrator for applying vibration-type stimulation to the skin.

The pressurizer may include a hollow holder fixed at one side of the stimulator fixing member; and a stimulating pin inserted into a hollow which is formed inside the hollow holder and moving forward and backward in the hollow, in which the front end portion is moved to pressurize the skin to provide stimulation, and the back end portion may include a pressing unit or an actuator.

The pressurizer may further include a raised portion formed on the stimulating pin to move together with the stimulating pin; and a stopper formed in the hollow, in which the raised portion is blocked by the stopper.

Unlike this, the stimulator may include a hollow holder fixed at one side of the stimulator fixing member and having multiple hollows; and at least one stimulating pin inserted into a hollow that is formed inside the hollow holder and moving forward and backward in the hollow, in which the front end portion is moved to pressurize the skin to provide stimulation and the back end portion may include a pressing unit or an actuator.

The stimulator may further include a holder fixing member for rotatably fixing the hollow holder to the simulator fixing member.

The holder fixing member is integrally fixed to the simulator fixing member and may include a fixing ring having an inner circumferential surface so as to allow the hollow holder to be fitted to the fixing ring.

The stimulator may include a toe moving member for moving the position of the toe to stimulate the toe.

The toe moving member may include a lift for lifting the toe of the feet; and an elevator for elevating the lift.

The stimulator may include an electric heater for applying heat to the skin to stimulate the skin.

The present invention may further include at least one blood circulation detecting sensor placed on the stimulator fixing member for detecting blood circulation of the human body.

The blood circulation detecting sensor may include at least one of an oxygen saturation sensor for measuring oxygen saturation in the blood inside the skin and a temperature measuring sensor for measuring skin temperature.

The simulator fixing member may include an elastic clothing that is wearable on the hands or feet of the human body.

The simulator fixing member may include a fixing frame corresponding to the hands or feet of the human body.

The fixing frame may include a supporting plate for supporting the hands or feet of the human body; and a first extendable rod extending to the upward direction of the supporting plate, inwhich the stimulator is integrally fixed to the first extendable rod.

The fixing frame may include a supporting plate for supporting the hands or feet of the human body; a first extendable rod extending to the upward direction of the supporting plate; a second extendable rod extending to the different direction from the first extendable rod and having the stimulator at the front end portion; and a joint for fixing the second extendable rod to the first extendable rod.

The simulator fixing member may further include a stimulator movement rail for guiding the path of movement of the stimulator.

The stimulator movement rail may have a rail groove corresponding to a protrusion formed on one side of the stimulator.

In the meanwhile, the present invention includes: a stimulator for stimulating the human body to examine nerve function of the human body, having at least one of a pressurizer for pressurizing the skin to provide stimulation, a vibrator for applying vibration-type stimulation to the skin, a toe moving member for moving the position of the toe to stimulate the toe, and an electric heater for applying heat to the skin to stimulate the skin; a stimulator fixing member formed in the shape corresponding to the hands or feet of the human body, in which the stimulator is located at one side so as to fix the stimulator to the hands or feet of the human body; a blood circulation detecting sensor for detecting blood circulation of the human body, placed on the stimulator fixing member and having at least one of an oxygen saturation sensor for measuring oxygen saturation in the blood inside the skin and a temperature measuring sensor for measuring skin temperature; a confirmation button operated by a subject to produce a confirmation signal which confirms that the skin or the toe has been stimulated by the stimulator; a terminal for setting a sequential measurement process by comparing a pre-set stepwise measurement process based on a confirmation signal produced by the confirmation button and a measurement value measured in the blood circulation detecting sensor, or for converting the confirmation signal or the measurement value into printable data; a guiding unit for guiding the sequential measurement process set in the terminal in an audio or video message; and a printer connected to the guiding unit through the terminal 51 so as to communicate with each other for printing out data converted in the terminal.

### EFFECT OF THE INVENTION

According the present invention, because nerve function is examined by checking if sensation is detected by the stimulator, the device for examining nerve function has the following effects: significantly improving precision and reliability of examinations for nerve function; and systemically and actively determining an exact diagnosis for patients having hypoesthesia with the symptoms of diabetes or spinal nerve damage; also exactly diagnosing the area of chronic neurological complications, and accurately adjusting the degree of stimulation; and recording and checking the degree of hypoesthesia in real time and for a long period of time with a subject wearing an elastic clothing, stimulation gloves or stimulation boots in practical life.

In particular, a systematic and scientific examination is possible because the device is capable of: checking if a subject can feel pain caused by applying pressure, and if a subject can distinguish positions between two pressure points (positions of two areas), and if a subject can detect vibration, if a subject can detect change in temperature, and if a subject can distinguish difference in the applied pressure, through the pain caused by the applied pressure; and checking the degree of hypoesthesia by screening the state of blood circulation through oxygen saturation or temperature of the hypoesthesia area. In addition, because the device is capable of not only guiding the stepwise process of measuring the capabilities by voice or display, it is possible to not only perform examinations with ease, but also to see the measured capabilities and the state of blood circulation in an audio or video message, or in a printed material so as to understand the degree of hypoesthesia with ease.

### BRIEF DESCRIPTION OF DRAWINGS

FIG. 1 is a cross-sectional view illustrating a device for examining nerve function according to the first embodiment of the present invention.

FIG. 2 is a cross-sectional view illustrating an example of the pressurizer illustrated in FIG. 1.

FIG. 3 is a cross-sectional view illustrating an example of the vibrator illustrated in FIG. 1.

FIG. 4 is a cross-sectional view illustrating an example of the movement rail illustrated in FIG. 1.

FIG. 5 is a cross-sectional view illustrating a device for examining nerve function according to the second embodiment of the present invention.

FIG. 6 is a cross-sectional view illustrating a first extendable rod, a second extendable rod and a joint of FIG. 5.

FIG. 7 is a cross-sectional view illustrating a device for examining nerve function according to the third embodiment of the present invention.

FIG. 8 is a cross-sectional view illustrating a device for examining nerve function according to the fourth embodiment of the present invention.

FIG. 9 is a cross-sectional view illustrating a device for examining nerve function according to the fifth embodiment of the present invention.

FIG. 10 is a cross-sectional view illustrating a device for examining nerve function according to the sixth embodiment of the present invention.

FIG. 11 is a cross-sectional view illustrating a device for examining nerve function according to the seventh embodiment of the present invention.

FIG. 12 is a cross-sectional view illustrating a device for examining nerve function according to the eighth embodiment of the present invention.

### BEST MODE(S) FOR CARRYING OUT THE INVENTION

Hereinafter, the device for examining nerve function according to preferred embodiments of the present invention will be described in detail with reference to the accompanying drawings.

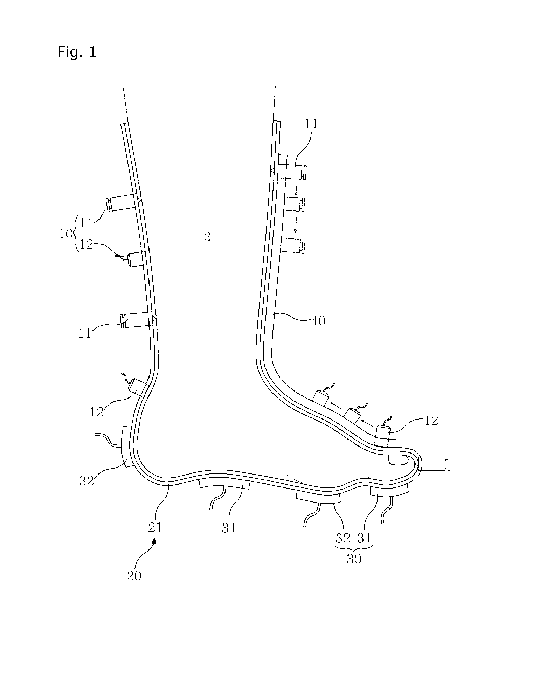

First, the device for examining nerve function according to the first embodiment of the present invention may include a stimulator 10 and a stimulator fixing member 20, as shown in FIGs. 1 to 4.

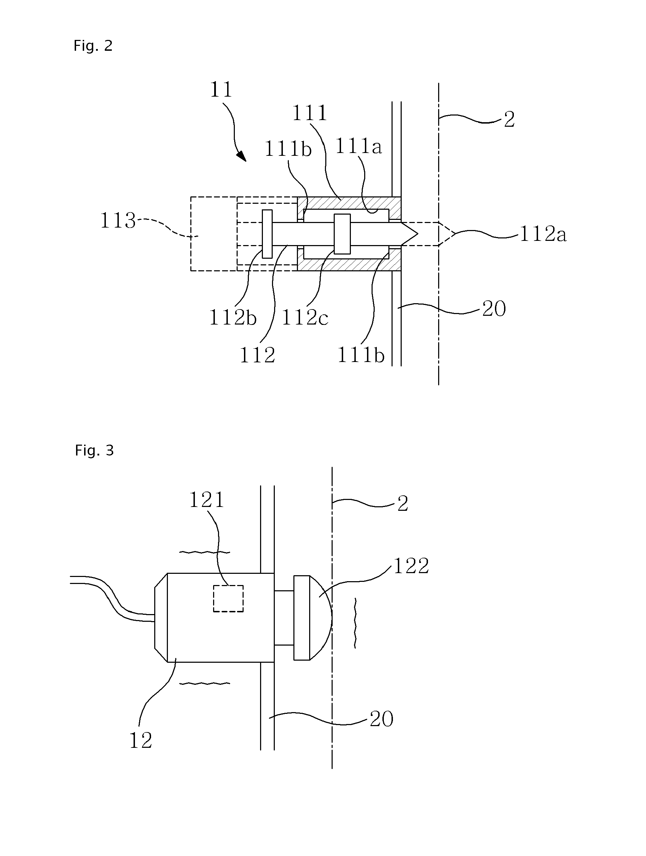

The stimulator 10, which generates stimulation on the human body in order to examine nerve function of the human body, includes a pressurizer 11 for pressurizing the skin with a stick-type stimulating pin 112 as shown in FIG. 2 and/or a vibrator 12 for applying vibration to the skin that comes into contact with a vibration surface 112 with a vibration motor 121 as shown in FIG. 3.

The pressurizer 11 may include a hollow holder 111 and a stimulating pin 112 as shown in FIG. 2.

The hollow holder 111 is fixed at one side of an elastic clothing 21, which is a kind of stimulator fixing member 20, and multiple hollow holders may be provided, for example at an important stimulation point such as a meridian point, at a main check point representing a long distance from the heart, or at a position representing a certain distance from the toes.

The stimulating pin 112 may be made of wood or of a monofilament of plastic material or nylon material. Such a stimulating pin 112 is inserted into a hollow 111a that is formed inside the hollow holder 111 in such a manner so as to be movable forward and backward.

The stimulating pin 112 may has a blunt front end portion, but it is rather preferable to form an actuate stimulation point 112a. Also, the back end portion may preferably include a pressing unit 112b or an actuator 13 for enabling forward and backward movement of the stimulating pin 112 in a selective manner. Such an actuator 13 can be configured to adjust the movement distance of the stimulating pin 112 which moves forward and backward depending on the magnitude of supplied voltage.

According to the present invention, inspectors may therefore check an accurate response from the subject's response when considering the numerical stimulation that is expressed in a certain distance from the toes of the subject, i.e. the length of forward movement of the stimulating pin 112 that is moved forward by the actuator 113. As a result, the device is capable of automatically and numerically measuring how much the symptoms of hypoesthesia have improved or deteriorated. Especially, in the case that the movement distance of the stimulating pin 112 is adjusted by the actuator 113, it is able to adjust the strength of the contact of the stimulating pin 112, thereby precisely checking the sensory reaction from the subject.

Meanwhile, the stimulating pin 112 described above may be connected to a sensor for detecting the movement distance when moving forward and backward, which is not illustrated. It is preferable that such a sensor is placed in the hollow holder 111 and may include a normal optical sensor or hall sensor. Those skilled in the art could easily understand the configuration of such a sensor, and thus its detailed description will be omitted.

On the other the hand, multiple pressurizers 11 may be provided with one adjacent to another and with one separated from another. These pressurizers 11 individually pressurize the skin in a state where they are separated from one another. In other words, the pressurizers 11 individually pressurize two areas at the same time. Therefore, the subject may check whether to distinguish two stimulation points at which the stimulation occurs. That is, if the subject is unable to distinguish two stimulation points, it may be determined that the sensation has been reduced at the areas corresponding to the stimulation points.

In the pressurizers 11, a raised portion 112c may be formed on the stimulating pin 112 so as to prevent the stimulating pin 112 that moves inside the hollow holder 111 from moving away from the hollow holder 111 as shown in FIG. 2, and a stopper 111b may be formed in the hollow 111a. That is, the raised portion 112c is blocked by the stopper 111b and thereby restricting the forward/backward distance of the stimulating pin 112.

Here, the hollow holder 111 described above, as illustrated, may be integrally fixed to the stimulator fixing member 20 via a movement rail 40, which will be described below. In other words, the hollow holder 111 may be indirectly fixed to the stimulator fixing member 20. Such a hollow holder 111 may be fixed to the movement rail 40 in a detachable manner by bolting, and it may also be permanently fixed to the movement rail 40 by welding or bonding.

Otherwise, the hollow holder 111 may be directly fixed to the stimulator fixing member 20. In this case, the hollow holder 111 is directly connected to the stimulator fixing member 20 by bolting or bonding. In such a case that the stimulator fixing member 20 is the elastic clothing 21 described above, the hollow holder 111 may be fixed to the elastic clothing 21 by bonding, or fixed to the elastic clothing 21 by bolting using an bracket, which is not illustrated. Of course, the vibrator described above is fixed to the stimulator fixing member 20, the movement rail 40 or the elastic clothing 21 in the same way as the hollow holder 111. Those skilled in the art could easily carry out the method of fixing such a hollow holder 111 and a vibrator 12, and thus its detailed description will be omitted

As shown in FIG. 1, the stimulator fixing member 20 may include multiple pressurizers 11 and multiple vibrators 12 at one side to fix the stimulator 10 to the hands 1 or the feet 2 of the human body, and it is formed in the shape corresponding to the feet 2 of the human body, and it may also include the elastic clothing 21 which is wearable on the feet 2.

In general, the elastic clothing 21 may include either a socks-type item of clothing or a stocking-type item of clothing, as shown in FIG. 1.

Accordingly, when using the device for examining nerve function according to the present invention, the subject may move in a state where the subject is wearing the elastic clothing 21 and at the same time check the degree of hypoesthesia by receiving various types of stimulation such as pressure or vibration.

In the meantime, the device for examining nerve function according to the present invention may further include a blood circulation detecting sensor 30 for detecting blood circulation of the human body, which is placed on the stimulator fixing member 20.

The blood circulation detecting sensor 30, which is a sensor for detecting blood circulation of the human body, may include an oxygen saturation sensor 31 for measuring oxygen saturation in the blood inside the skin and/or a temperature measuring sensor 32 for measuring skin temperature, as shown in FIG. 1.

Accordingly, when using the device for examining nerve function according to the present invention, the subject may move in a state where the subject is wearing the elastic clothing 21 and at the same time check the patient's nerve function by measuring oxygen saturation in the blood circulating in the feet or feet temperature.

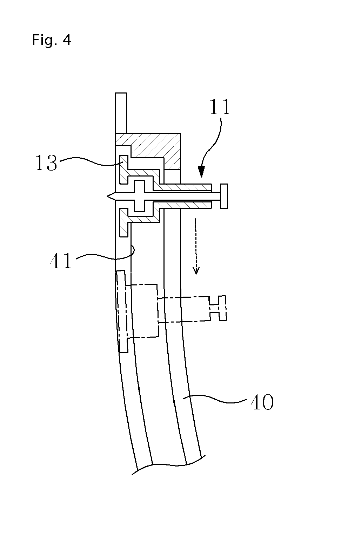

In the meanwhile, it is also possible to provide a stimulator movement rail 40 on the stimulator fixing member 20 for guiding the path of movement of the stimulator 10 so as to reduce the number of pressurizers 11 or vibrators 12 arranged on the feet of the subject and to provide stimulation to one point or to some points contiously, as shown in FIG. 4.

Here, the stimulator movement rail 40 has a rail groove 41 formed to correspond to a protrusion 13 that is formed on one side of the pressurizer 11 or the vibrator 12 as shown in FIG. 4, so that the pressurizer 11 or the vibrator 12 could apply stimulation to specific points or to a specific point or to some points continuously moving along the rail groove 41.

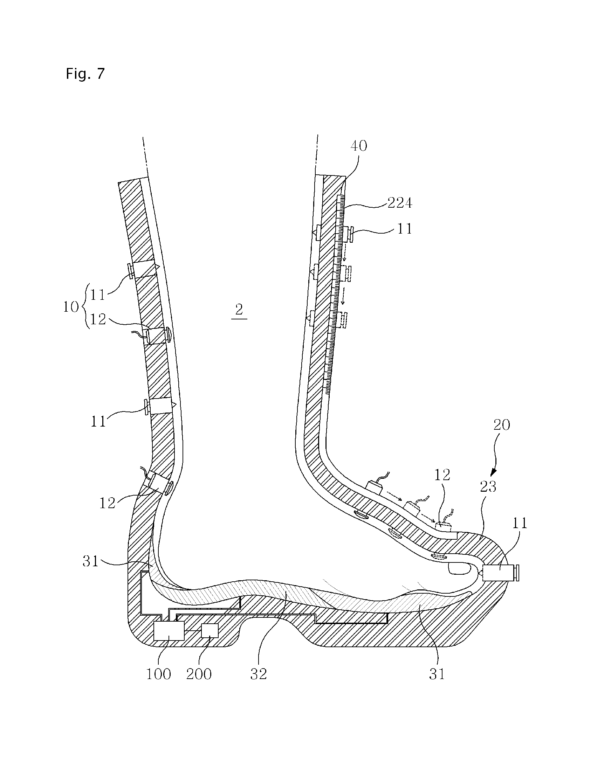

As shown in FIG. 7, the stimulator movement rail 40 may be provided on sensitive boots 23, which will be described below, and in this case, a scaled ruler 224 may be placed at one side so as to measure the movement distance, height, etc. of the stimulator 10.

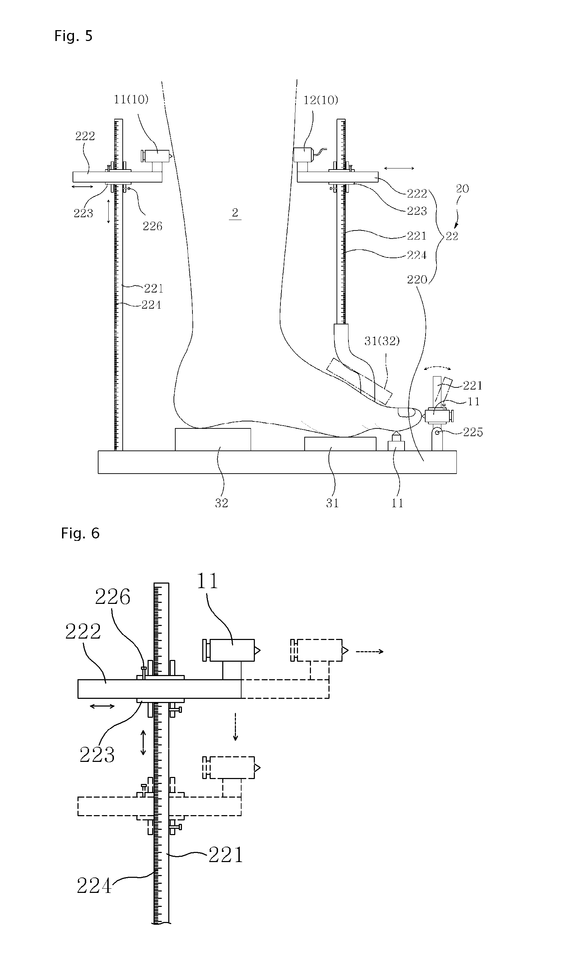

As shown in FIGs. 5 and 6, the device for examining nerve function according to the second embodiment of the present invention may include a fixing frame 22 corresponding to the feet 2 of the human body, as a kind of stimulator fixing member 20.

The fixing frame 22 includes a supporting plate 220, a first extendable rod 221, a second extendable rod 222 and a joint 223, as shown in FIG. 5.

The supporting plate 220 supports the feet 2 of the human body. When the subject puts the feet on the supporting plate 220, the oxygen saturation sensor 31 and the temperature measuring sensor 32 placed on the supporting plate 220 may check blood condition of the patent's feet. The oxygen saturation sensor 31 and the temperature measuring sensor 32 of the supporting plate 220 may also be placed at the first extendable rod 221 so that they may operate above the supporting plate 220, as marked in a broken line in the figure. That is, the oxygen saturation sensor 31 and the temperature measuring sensor 32 may be either placed on the first extendable rod 221 so as to measure the upper portion of the feet 2, or placed on the supporting plate 220 so as to measure the bottom surface of the feet 2.

The first extendable rod 221 is placed in such a manner as to extend above the supporting plate 220, as shown in FIG. 5. If there are scale marks 224 for measuring the height of the pressurizer 11 or the vibrator 12, the measurement position will be expressed in a number.

In addition, the first extendable rod 221 is hinge-connected to a hinge shaft 225 placed on the supporting plate 220. Before the subject puts the feet on the supporting plate 220, the first extendable rod 221 remains splayed out. When the subject puts the feet on the supporting plate 220, however, the first extendable rod 221 stands up to the direction of the feet, resulting in easier sensation examination.

The second extendable rod 222 is placed in such a manner as to extend to the different direction from the first extendable rod 221, in which the pressurizer 11 or the vibrator is placed at the front end portion of the second extendable rod. At this time, it is preferable that the second extendable rod 222 is placed in such a manner as to extend to the perpendicular direction with regard to the first extendable rod 221. Here, it is possible not to provide the second extendable rod 222 when the pressurizer 11 or the vibrator 12 is placed on the first extendable rod 221. In other words, the pressurizer 11 or the vibrator 12 may be placed on the first extendable rod 221. The pressurizer 11 or the vibrator 12 may be embedded into the supporting plate 220.

The joint 223 fixes the second extendable rod 222 to the first extendable rod 221. Here, the joint 223, which fixes the first extendable rod 221 and the second extendable rod 222 to each other using a fixing unit such as a clamping screw 226, may freely adjust the horizontal position and vertical position of the stimulator 10, as shown in FIG. 6.

As shown in FIG. 7, the device for examining nerve function according to the third embodiment of the present invention may include sensitive boots 23 which are wearable on the feet 2 of the human body, as a kind of stimulator fixing member 20.

The sensitive boots 23 may include a pressurizer 11, a vibrator 12, an oxygen saturation sensor 31 and a temperature measuring sensor 32, all of which were described above.

Further, the sensitive boots 23 may include a data storage unit 100 for storing measurement values measured in the oxygen saturation sensor 31 and temperature measuring sensor 32, so that the subject can measure changes in oxygen saturation and temperature of the feet 2 in practical life for a long period of time, and a battery 200 for supplying power to the stimulator 10 and the data storage unit 100.

Accordingly, the subject can record and check the degree of hypoesthesia in real time and for a long period of time, wearing the sensitive boots 23 in practical life.

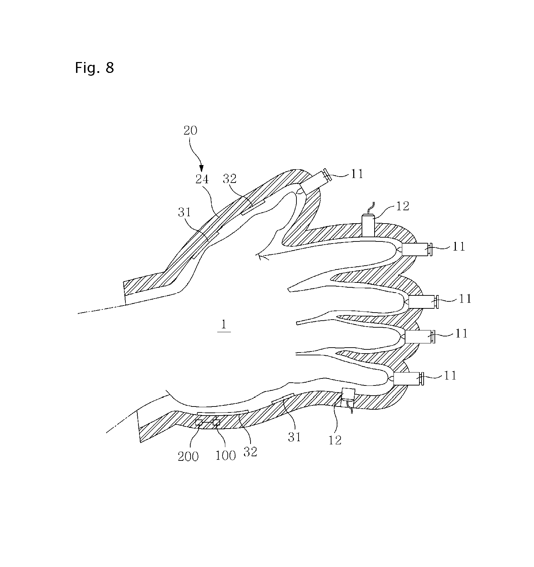

As shown in FIG. 8, the device for examining nerve function according to the fourth embodiment of the present invention may include sensitive gloves 24 which are wearable on the hands 1 of the human body, as a kind of stimulator fixing member 20.

The sensitive gloves 24 may include a pressurizer 11, a vibrator 12, an oxygen saturation sensor 31 and a temperature measuring sensor 32, all of which were described above.

Further, the sensitive gloves 24 may include a data storage unit 100 for storing measurement values measured in the oxygen saturation sensor 31 and temperature measuring sensor 32, so that the subject can measure changes in oxygen saturation and temperature of the hands 1 in practical life for a long period of time, and a battery 200 for supplying power to the stimulator 10 and the data storage unit 100.

Accordingly, the subject can record and check the degree of hypoesthesia in real time and for a long period of time, wearing the sensitive gloves 24 in practical life.

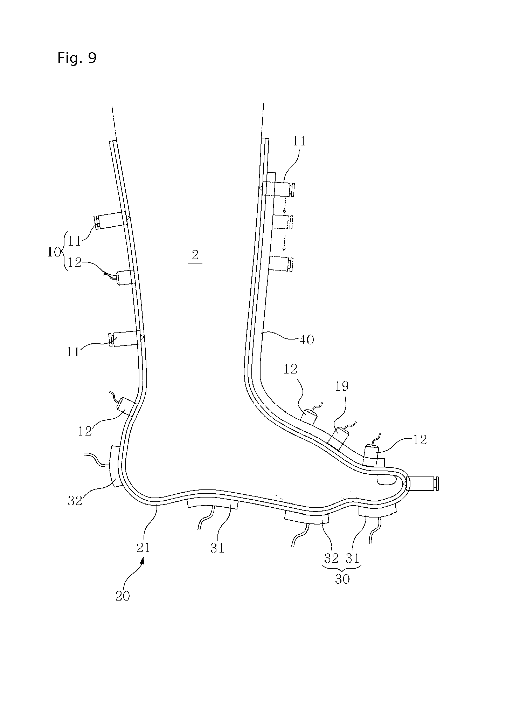

As shown in FIG. 9, the device for examining nerve function according to the fifth embodiment of the present invention has the same configuration as the device according to the first embodiment, except that there is an only difference from the first embodiment in that the stimulator includes an electric heater 19. This electric heater 19 generates heat with an internal electric coil (not illustrated) to stimulate the skin through the heat by applying the heat of the electric coil to the skin. Therefore, the subject may detect the stimulation by the heat and check the area of hypoesthesia. That is, the subject may determine that the sensation is not reduced on the area where the electric heater 19 is located if the subject feels the heat from the electric heater 19. Otherwise, it may be determined that the sensation has been reduced.

The electric heater 19 may be placed on the stimulator fixing member 20 together with the pressurizer 11 and the vibrator 12 described above, but it may also be placed on the stimulator fixing member 20 alone. When the electric heater 19 is arranged in the above-described movement rail 40 illustrated in FIG. 4, it may move along the movement rail 40.

The configuration of the electric heater 19 could easily be not only understood but also embodied by those skilled in the art, and thus its detailed description will be omitted.

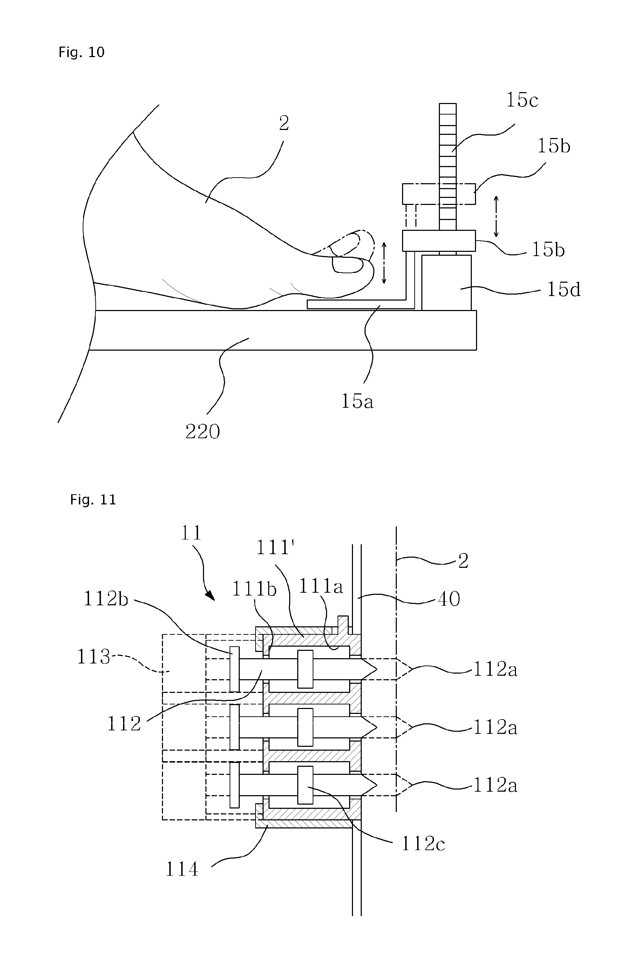

As shown in FIG. 10, the device for examining nerve function according to the sixth embodiment of the present invention is different from the device according to the embodiment described above only in that the stimulator includes a toe moving member 15. The toe moving member 15 elevates the toe to move the position of the toe.

The toe moving member 15, for example, may include a lift 15a for lifting the toe and an elevator for elevating the lift 15a, as illustrated.

The lift 15a may be formed either in the shape of the plate so as to support a lower portion of the toe as illustrated, or in a cylinder so as to allow the toe to be inserted.

The elevator may include a rotating motor 15d, a pivot shaft 15c which is pivotally rotated by the rotating motor 15d on the supporting plate, and an elevation nut 15b that is screw-connected to the pivot shaft 15c to be elevated and connected to the lift 15a. In other words, the toe moving member 15 may include a lift 15a, a rotating motor 15d, a pivot shaft 15c and an elevation nut 15b.

The pivot shaft 15c is pivotally rotated by the rotating motor 15d to elevate the lift 15a along with the elevation nut 15b, thereby moving the position of the toe. As a result, the subject may check the area of the toe where the sensation is reduced through the stimulation to the toe produced by the movement of the position. That is, the subject may determine that the sensation of the examined toe is not reduced if the subject detects the stimulation by the movement of the position. Otherwise, it may be determined that the sensation of the examined toe has been reduced.

As shown in FIG. 11, the device for examining nerve function according to the seventh embodiment of the present invention is configured in a different manner from the above-described ones in the pressurizer 11. Such a pressurizer 11, for example, may include a hollow holder 111' fixed to the stimulator fixing member 20 described above through the movement rail 40 and having multiple hollows 111a, and at least one stimulating pin 112 inserted into a hollow 111a that is formed inside the hollow holder 111' and moves forward and backward in the hollow, in which the front end portion is moved to pressurize the skin to provide stimulation and the back end portion comprises a pressing unit 112b or an actuator 113, as illustrated. At this time, the hollow holder 111' is fixed at one end of the movement rail 40 to be connected to the stimulator fixing member 20 described above.

Here, the pressurizer 11 described above may further include a holder fixing member for rotatably fixing the hollow holder 111' to the movement rail 40 on the stimulator fixing member 20 described above. The holder fixing member, for example, is integrally connected to the movement rail 40 on the stimulator fixing member 20 described above, and may include a fixing ring having an inner circumferential surface so as to allow the hollow holder to be fitted to the fixing ring. In other words, the hollow holder 111' is mounted into the fixing ring 114 to be fixed to the movement rail 40, thereby being rotatably fixed to the movement rail 40.

The fixing ring 114 may be fixed to the movement rail 40 in a detachable manner by bolting, or permanently fixed to the movement rail 40 by welding or bonding. Such a fixing ring 114 preferably has an end portion which is formed in such a manner as to be bent inwards as illustrated. Therefore, the bent end portion allows the fixing ring 114 to rotatably bind the hollow holder 111'.

Meanwhile, the stimulating pins 112 comes in various thickness and each is inserted into one of multiple hollows 111a. That is, the stimulating pins 112 include multiple pins which are different from each other in thickness. Therefore, only one of the stimulating pins 112 comes out of the hollow holder 111' to stimulate the skin as needed. When the hollow holder 111' rotates in the same way as the revolver in the gun, the stimulating pins 112 come out one by one based on their thickness to stimulate the skin. In other words, the inspector may examine the subject's skin in various ways.

On the other the hand, in the case that the stimulation fixing member 20 includes the above-described fixing frame 22 illustrated in FIG. 5, the hollow holder 111' or the fixing ring 114 described above is placed on the fixing frame 22. Of course, the hollow holder 111' or the fixing ring 114 is fixed to the first extendable rod 221 or the second extendable rod 222 of the fixing frame 22. At this time, the hollow holder 111' or the fixing ring 114 is preferably fixed to the first extendable rod 221 or the second extendable rod 222 via the joint 223 described above.

Also, the device for examining nerve function according to the first to sixth embodiments of the present invention described above may be configured to examine a patient in a state where the patient is lying. This could be easily understood by those skilled in the art, and thus its detailed description will be omitted.

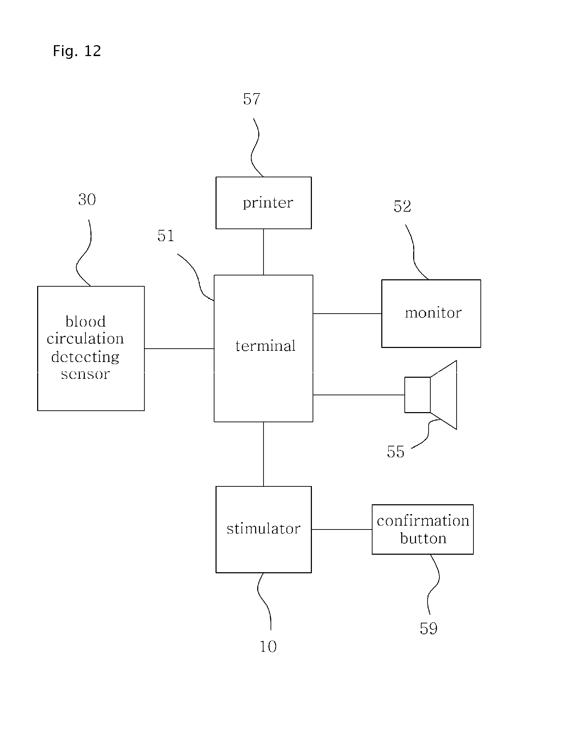

As shown in FIG. 12, the device for examining nerve function according to the eighth embodiment of the present includes a stimulator 10, a stimulator fixing member 20, a blood circulation detecting sensor 30, a confirmation button 59, a terminal 51, a printer 57, and a guiding unit.

The stimulator 10 includes at least one of a pressurizer 11, a vibrator 14, a toe moving member 15, and an electric heater 19, all of which were described above. And the stimulator fixing member 20 includes an elastic clothing 21 or a fixing frame 22, both of which were described above. Further, the blood circulation detecting sensor 30 includes an oxygen saturation sensor 31 and/or a temperature measuring sensor 32, both of which were described above. In addition, the terminal 51 may include a common personal computer (PC). Also, the guiding unit may include, for example, a monitor 52 or a speaker 55 as illustrated.

The confirmation button 59 is connected to the terminal 51. When the subject operates the confirmation button, the resultant confirmation signal is transmitted to the terminal 51.

In the device for examining nerve function according to the eighth embodiment of the present invention, a confirmation signal is transmitted to the terminal 51 by the confirmation button 59 when either the skin or the toe is stimulated by the stimulator 10 and the subject operates the confirmation button 59, and the measurement value that is measured in the blood circulation detecting sensor 30 is also transmitted to the terminal 51. Therefore, the terminal 51 sets a sequential measurement process by comparing the pre-set stepwise measurement process based on the confirmation signal produced by operating the confirmation button and the measurement value measured in the blood circulation detecting sensor 30, or converts the confirmation signal or the measurement value into printable data. The terminal 51 sets the sequential measurement process through an installed drive program. Also, the terminal 51 is capable of storing the printable data in a file, transmitting the data online, or storing the data on a storage medium in a CD type. In addition, the terminal may inform the patient's condition, based on the confirmation signal produced by the confirmation button, through a guiding unit. This could be easily understood or embodied by those skilled in the art, and thus its detailed description will be omitted.

Here, the pre-set stepwise measurement process described above refers to the order of the stepwise operation of the stimulator 10. That is, the stepwise measurement process is a process of performing many different types of examinations in order using the multiple types of stimulators 10 including a pressurizer 11, a vibrator 14, a toe moving member 15, and an electric heater 19. In more detail, the stepwise measurement process may include the following steps in order: pressurizing the skin with the pressurizer 10; and then vibrating the skin with the vibrator 14; and thereafter stimulating the toe with the two moving member 15, and finally stimulating the skin with the electric heater 19.

In the meanwhile, the terminal 51 guides the stepwise measurement process in a video or audio message via a monitor 52 or a speaker 55. Once the stimulation by the pressurizer 11 is complete, the terminal 51 informs via a monitor 52 or a speaker 55 that another examination will be carried out by the vibrator 14. At this time, the terminal 51 is confirmed that the stimulation by the pressurizer 11 has been complete through a confirmation signal produced by the confirmation button 59. And then, the terminal 51 informs via a monitor 52 or a speaker 55 that another examination will be carried out by the toe moving member 15 once it is confirmed by the confirmation button 59 that the stimulation by the vibrator 14 has been complete. The terminal 51 guides patients in such a way until the final examination by the electric heater 19 is complete.

The printer 57 may be connected to the guiding unit through the terminal 51 so as to communicate with each other. The printer 57 prints out the data converted in the terminal 51. Accordingly, the inspector may see the subject's examination results at a single look.

The present invention is not limited to the above-described embodiments, and various modifications and changes can be made by those skilled in the art without departing from the spirit and scope of the present invention. It is therefore intended that the claims cover all such modifications and other changes encompassed by the accompanying claims, not by the description.

### INDUSTRIAL APPLICABILITY

The device for examining nerve function according to the present invention can check if the sensation is detected by a stimulator to examine nerve function, and it is therefore capable of greatly improving accuracy and reliability of examinations for nerve function.

## Claims

1. A device for examining nerve function comprising:
at least one stimulator for stimulating the human body to examine nerve function of the human body; and
a stimulator fixing member formed in the shape corresponding to the hands or feet of the human body, wherein the stimulator is located at one side of the stimulator fixing member so as to fix the stimulator to the hands or feet of the human body.

2. The device according to claim 1, wherein the stimulator comprises at least one of a pressurizer for pressurizing the skin to provide stimulation and a vibrator for applying vibration-type stimulation to the skin.

3. The device according to claim 2, wherein the pressurizer comprises a hollow holder fixed at one side of the stimulator fixing member; and a stimulating pin inserted into a hollow which is formed inside the hollow holder and moving forward and backward in the hollow, wherein the front end portion of the stimulating pin is moved to pressurize the skin to provide stimulation, and the back end portion comprises a pressing unit or an actuator.

4. The device according to claim 3, wherein the pressurizer further comprises a raised portion formed on the stimulating pin and moving together with the stimulating pin; a stopper formed in the hollow, wherein the raised portion is blocked by the stopper.

5. The device according to claim 2, wherein the stimulator comprises a hollow holder fixed at one side of the stimulator fixing member and having a plurality of hollows; and at least one stimulating pin inserted into a hollow which is formed inside the hollow holder and moving forward and backward in the hollow, wherein the front end portion of the stimulation pin is moved to pressurize the skin to provide stimulation, and the back end portion comprises a pressing unit or an actuator.

6. The device according to claim 5, wherein the stimulator further comprises a holder fixing member for rotatably fixing the hollow holder to the stimulator fixing member, wherein the holder fixing member is integrally fixed to the stimulator fixing member and comprises a fixing ring having an inner circumferential surface so as to allow the hollow holder to be fitted to the fixing ring.

7. The device according to claim 1, wherein the stimulator is a toe moving member for moving the position of the toe to stimulate the toe, wherein the toe moving member comprises a lift for lifting the toe of the feet; and an elevator for elevating the lift.

8. The device according to claim 1, wherein the stimulator is an electric heater for applying heat to the skin to stimulate the skin.

9. The device according to claim 1, further comprising at least one blood circulation detecting sensor placed on the stimulator fixing member for detecting blood circulation of the human body.

10. The device according to claim 9, wherein the blood circulation detecting sensor comprises at least one of an oxygen saturation sensor for measuring oxygen saturation in the blood inside the skin and a temperature measuring sensor for measuring skin temperature.

11. The device according to claim 1, wherein the stimulator fixing member is an elastic clothing wearable on the hands or the feet of the human body.

12. The device according to claim 1, wherein the stimulator fixing member is a fixing frame corresponding to the hands or the feet of the human body.

13. The device according to claim 12, wherein the fixing frame comprises a supporting plate for supporting the hands or the feet of the human body; and a first extendable rod extending to the upward direction of the supporting plate, wherein the stimulator is integrally fixed to the first extendable rod.

14. The device according to claim 12, wherein the fixing frame comprises a supporting plate for supporting the hands or the feet of the human body; a first extendable rod extending to the upward direction of the supporting plate; a second extendable rod extending to the different direction from the first extendable rod, wherein the stimulator is placed at the front end portion of the second extendable rod; and a joint for fixing the second extendable rod to the first extendable rod.

15. The device according to any one of claims 1 to 14, wherein the stimulator fixing member further comprises a stimulator movement rail for guiding the path of movement of the stimulator.

16. The device according to claim 15, wherein the stimulator movement rail has a rail groove corresponding to a protrusion formed on one side of the stimulator.

17. A device for examining nerve function comprising:
a stimulator for stimulating the human body to examine nerve function of the human body, having at least one of a pressurizer for pressurizing the skin to provide stimulation, a vibrator for applying vibration-type stimulator to the skin, a toe moving member for moving the position of the toe to stimulate the toe, and an electric heater for applying heat to the skin to stimulate the skin;
a stimulator fixing member formed in the shape corresponding to the hands or feet of the human body, wherein the stimulator is located at one side of the stimulator fixing member so as to fix the stimulator to the hands or feet of the human body.
a blood circulation detecting sensor for detecting blood circulation of the human body, placed on the stimulator fixing member and having at least one of an oxygen saturation sensor for measuring oxygen saturation in the blood inside the skin, and a temperature measuring sensor for measuring skin temperature;;
a confirmation button operated by a subject to produce a confirmation signal which confirms that the skin or the toe has been stimulated by the stimulator;
a terminal for setting a sequential measurement process by comparing a pre-set stepwise measurement process based on a confirmation signal produced by the confirmation button and a measurement value measured in the blood circulation detecting sensor, or for converting the confirmation signal or the measurement value into printable data;
a guiding unit for guiding the sequential measurement process set in the terminal in an audio or video message; and
a printer connected to the guiding unit through the terminal 51 so as to communicate with each other for printing out the data converted in the terminal.
